# EUROPEAN PATENT APPLICATION

(11) **EP 2 674 498 A1**
(43) Date of publication of application: **18.12.2013**
(21) Application number: 12172059.3
(22) Date of filing: 14.06.2012
(51) Int. Cl.: C12P 7/22, C12N 9/88, A23L 1/03, A61K 8/34, A61K 31/05

(54) **Asymmetric hydration of 4-hydroxystyrene derivatives employing decarboxylases**

(71) Applicant: ACIB GmbH, 8010 Graz (AT); Karl-Franzens-Universität, 8010 Graz (AT)
(72) Inventor: Wuensch, Christiane, 9500 Villach (AT); Gross, Johannes, 8010 Graz (AT); Glueck, Silvia M., 8130 Frohnleiten (AT); Faber, Kurt, 8042 Graz (AT)
(74) Representative: Gassner, Birgitta

(57) **Abstract**

A promiscuous catalytic hydratase-activity of phenolic acid decarboxylases allows the asymmetric addition of H₂O across the C=C bond of hydroxystyrene derivatives yielding (S)-4-(1-hydroxyethyl)-phenols in up to 82% conversion and 77% e.e.

SEQUENCE LISTING

<110>  ACIB GmbH

<120>  Asymmetric Enzymatic Hydration of Hydroxystyrene Derivatives

<130>  AC001EP

<160>  8

<170>  PatentIn version 3.5

<210>  1
<211>  276
<212>  PRT
<213>  Artificial Sequence

<220>
<223>  Codon-optimised sequence of HCHL

<400>  1

Met Ser Thr Tyr Glu Gly Arg Trp Lys Thr Val Lys Val Glu Ile Glu
1               5                   10                  15

Asp Gly Ile Ala Phe Val Ile Leu Asn Arg Pro Glu Lys Arg Asn Ala
            20                  25                  30

Met Ser Pro Thr Leu Asn Arg Glu Met Ile Asp Val Leu Glu Thr Leu
        35                  40                  45

Glu Gln Asp Pro Ala Ala Gly Val Leu Val Leu Thr Gly Ala Gly Glu
    50                  55                  60

Ala Trp Thr Ala Gly Met Asp Leu Lys Glu Tyr Phe Arg Glu Val Asp
65                  70                  75                  80

Ala Gly Pro Glu Ile Leu Gln Glu Lys Ile Arg Arg Glu Ala Ser Gln
                85                  90                  95

Trp Gln Trp Lys Leu Leu Arg Met Tyr Ala Lys Pro Thr Ile Ala Met
            100                 105                 110

Val Asn Gly Trp Cys Phe Gly Gly Gly Phe Ser Pro Leu Val Ala Cys
        115                 120                 125

Asp Leu Ala Ile Cys Ala Asp Glu Ala Thr Phe Gly Leu Ser Glu Ile
    130                 135                 140

Asn Trp Gly Ile Pro Pro Gly Asn Leu Val Ser Lys Ala Met Ala Asp
145                 150                 155                 160

Thr Val Gly His Arg Gln Ser Leu Tyr Tyr Ile Met Thr Gly Lys Thr
                165                 170                 175

Phe Gly Gly Gln Lys Ala Ala Glu Met Gly Leu Val Asn Glu Ser Val
            180                 185                 190

Pro Leu Ala Gln Leu Arg Glu Val Thr Ile Glu Leu Ala Arg Asn Leu
        195                 200                 205

Leu Glu Lys Asn Pro Val Val Leu Arg Ala Ala Lys His Gly Phe Lys
    210                 215                 220

Arg Cys Arg Glu Leu Thr Trp Glu Gln Asn Glu Asp Tyr Leu Tyr Ala
225                 230                 235                 240

Lys Leu Asp Gln Ser Arg Leu Leu Asp Thr Glu Gly Gly Arg Glu Gln
                245                 250                 255

Gly Met Lys Gln Phe Leu Asp Asp Lys Ser Ile Lys Pro Gly Leu Gln
            260                 265                 270

Ala Tyr Lys Arg
        275

<210>  2
<211>  162
<212>  PRT
<213>  Artificial Sequence

<220>
<223>  Codon-opimised sequence of PAD_Ba

<400>  2

Met Glu Asn Phe Ile Gly Ser His Met Ile Tyr Thr Tyr Glu Asn Gly
1               5                   10                  15

Trp Glu Tyr Glu Ile Tyr Ile Lys Asn Asp His Thr Ile Asp Tyr Arg
            20                  25                  30

Ile His Ser Gly Met Val Gly Gly Arg Trp Val Arg Asp Gln Glu Val
        35                  40                  45

Asn Ile Val Lys Leu Thr Glu Gly Val Tyr Lys Val Ser Trp Thr Glu
    50                  55                  60

Pro Thr Gly Thr Asp Val Ser Leu Asn Phe Met Pro Asn Glu Lys Arg
65                  70                  75                  80

Met His Gly Ile Ile Phe Phe Pro Lys Trp Val His Glu His Pro Glu
                85                  90                  95

Ile Thr Val Cys Tyr Gln Asn Asp Tyr Ile Asp Val Met Lys Glu Ser
            100                 105                 110

Arg Glu Lys Tyr Asp Thr Tyr Pro Lys Tyr Val Val Pro Glu Phe Ala
        115                 120                 125

Asp Ile Thr Tyr Leu Asn Asn Ala Gly Ile Asn Asn Glu Ala Leu Ile
    130                 135                 140

Ser Glu Ala Pro Tyr Glu Gly Met Thr Asp Asp Ile Arg Ala Gly Lys
145                 150                 155                 160

Leu Lys

<210>  3
<211>  178
<212>  PRT
<213>  Artificial Sequence

<220>
<223>  Codon-optimised sequence of PAD_Lp

<400>  3

Met Thr Lys Thr Phe Lys Thr Leu Asp Asp Phe Leu Gly Thr His Phe
1               5                   10                  15

Ile Tyr Thr Tyr Asp Asn Gly Trp Glu Tyr Glu Trp Tyr Ala Lys Asn
            20                  25                  30

Asp His Thr Val Asp Tyr Arg Ile His Gly Gly Met Val Ala Gly Arg
        35                  40                  45

Trp Val Thr Asp Gln Lys Ala Asp Ile Val Met Leu Thr Glu Gly Ile
    50                  55                  60

Tyr Lys Ile Ser Trp Thr Glu Pro Thr Gly Thr Asp Val Ala Leu Asp
65                  70                  75                  80

Phe Met Pro Asn Glu Lys Lys Leu His Gly Thr Ile Phe Phe Pro Lys
                85                  90                  95

Trp Val Glu Glu His Pro Glu Ile Thr Val Thr Tyr Gln Asn Glu His
            100                 105                 110

Ile Asp Leu Met Glu Gln Ser Arg Glu Lys Tyr Ala Thr Tyr Pro Lys
        115                 120                 125

Leu Val Val Pro Glu Phe Ala Asn Ile Thr Tyr Met Gly Asp Ala Gly
    130                 135                 140

Gln Asn Asn Glu Asp Val Ile Ser Glu Ala Pro Tyr Lys Glu Met Pro

145                 150                 155                 160

Asn Asp Ile Arg Asn Gly Lys Tyr Phe Asp Gln Asn Tyr His Arg Leu
                165                 170                 175

Asn Lys

<210>  4
<211>  168
<212>  PRT
<213>  Artificial Sequence

<220>
<223>  Codon-optimised sequence of FDC_Esp

<400>  4

Met Asn Thr Phe Asp Lys His Asp Leu Ser Gly Phe Val Gly Lys His
1               5                   10                  15

Leu Val Tyr Thr Tyr Asp Asn Gly Trp Asn Tyr Glu Ile Tyr Val Lys
            20                  25                  30

Asn Asp Asn Thr Ile Asp Tyr Arg Ile His Ser Gly Leu Val Gly Asn
        35                  40                  45

Arg Trp Val Lys Asp Gln Glu Ala Tyr Ile Val Arg Val Gly Glu Ser
    50                  55                  60

Ile Tyr Lys Ile Ser Trp Thr Glu Pro Thr Gly Thr Asp Val Ser Leu
65                  70                  75                  80

Ile Val Asn Leu Gly Asp Ser Leu Phe His Gly Thr Ile Phe Phe Pro
                85                  90                  95

Arg Trp Val Met Asn Asn Pro Glu Pro Thr Val Cys Phe Gln Asn Asp
            100                 105                 110

His Ile Pro Leu Met Asn Ser Tyr Arg Glu Ala Gly Pro Ala Tyr Pro
        115                 120                 125

Thr Glu Val Ile Asp Glu Phe Ala Thr Ile Thr Phe Val Arg Asp Cys
    130                 135                 140

Gly Ala Asn Asn Glu Ser Val Ile Ala Cys Ala Ala Ser Glu Leu Pro
145                 150                 155                 160

Lys Asn Phe Pro Asp Asn Leu Lys
                165

<210>  5
<211>  169
<212>  PRT
<213>  Artificial Sequence

<220>
<223>  Codon-optimised sequence of PAD_Psp

<400>  5

Met Ser Thr Phe Asp Lys His Asp Leu Ser Gly Ile Val Gly Lys His
1               5                   10                  15

Leu Val Tyr Thr Tyr Asp Asn Gly Trp Asn Tyr Glu Leu Tyr Ile Lys
            20                  25                  30

Asn Ala Ser Thr Ile Asp Tyr Arg Ile His Ser Gly Met Val Gly Asn
        35                  40                  45

Arg Trp Val Lys Asn Gln His Val Tyr Val Val Arg Leu Ala Gln Asp
    50                  55                  60

Val Tyr Lys Val Ser Trp Thr Glu Pro Thr Gly Thr Asp Val Ser Leu
65                  70                  75                  80

Ala Val Asn Leu Ala Asp Lys Ile Phe His Gly Thr Ile Phe Phe Pro
                85                  90                  95

Arg Trp Val Met Asn Asn Pro Glu Lys Thr Val Cys Phe Gln Asn Asp
            100                 105                 110

His Leu Glu Glu Met Ala Lys Phe Arg Glu Thr Gly Pro Ala Tyr Pro
        115                 120                 125

Thr Glu Ile Ile Asp Glu Phe Ala Thr Leu Thr Val Ile Arg Glu Val
    130                 135                 140

Gly Glu Asn Asn Glu Ser Val Ile Asp Cys Pro Pro Asp Gln Leu Pro
145                 150                 155                 160

Ala Asn Trp Pro Ala Asn Leu Gln Asn
                165

<210>  6
<211>  177
<212>  PRT
<213>  Artificial Sequence

<220>
<223>  Codon-optimised sequence of PAD_Ll

<400>  6

Met Lys Thr Phe Lys Ser Leu Glu Asp Phe Val Gly Thr His Phe Ile

1               5                   10                  15

Tyr Thr Tyr Asp Asn Gly Trp Glu Tyr Glu Leu Tyr Val Lys Asn Asp
            20                  25                  30

His Thr Ile Asp Tyr Arg Ile His Gly Gly Met Val Ala Gly Arg Trp
        35                  40                  45

Val Lys Asp Gln Glu Val Ser Leu Val Met Leu Thr Glu Gly Ile Tyr
    50                  55                  60

Lys Ile Thr Trp Thr Glu Pro Thr Gly Thr Asp Val Ala Leu Asp Phe
65                  70                  75                  80

Leu Pro Asn Glu Gly Lys Leu His Gly Met Ile Phe Phe Pro Lys Trp
                85                  90                  95

Val Glu Glu His Pro Glu Ile Thr Val Cys Phe Gln Asn Asp Phe Ile
            100                 105                 110

Asp Leu Met His Glu Ser Arg Glu Lys Tyr Glu Thr Tyr Pro Lys Tyr
        115                 120                 125

Leu Val Pro Glu Phe Ala Lys Ile Thr Tyr Ala Ala Glu Ala Gly Lys
    130                 135                 140

Asp Asn Asp Asp Val Val Ala Gln Ala Pro Tyr Lys Glu Met Thr Asn
145                 150                 155                 160

Asp Ile Arg Asn Gly Lys Tyr Phe Asp Gln Asn Tyr Lys Met Ile Lys
                165                 170                 175

His

<210>  7
<211>  172
<212>  PRT
<213>  Artificial Sequence

<220>
<223>  Codon-optimised sequence of PAD_Mc

<400>  7

Met Thr Ser Val Thr Asn Pro Ile Pro Pro Gln Asp Leu Ser Gly Ile
1               5                   10                  15

Val Gly His Arg Phe Ile Tyr Thr Tyr Ala Asn Gly Trp Gln Tyr Glu
            20                  25                  30

Met Tyr Val Lys Asn Val Thr Thr Ile Asp Tyr Arg Ile His Ser Gly
        35                  40                  45

His Val Gly Gly Arg Trp Val Lys Gly Gln Gln Val Asn Leu Val Gln
    50                  55                  60

Leu Asp Asp Asp Ser Phe Lys Ile Ser Trp Thr Glu Pro Thr Gly Thr
65                  70                  75                  80

Cys Val Ala Val Asn Val Leu Pro Gly Lys Arg Arg Ile His Gly Val
                85                  90                  95

Ile Phe Phe Pro Gln Trp Ile Arg Met His Gly Glu His Thr Val Cys
            100                 105                 110

Phe Gln Asn Asp His Leu Asp Glu Met Arg Ala Tyr Arg Asp Arg Gly
        115                 120                 125

Pro Thr Tyr Pro Ile Tyr Glu Val Pro Glu Phe Ala Tyr Ile Thr Leu
    130                 135                 140

Phe Glu Tyr Val Gly Thr Asp Asp Glu Thr Val Ile Asp Thr Gly Pro
145                 150                 155                 160

Glu His Leu Pro Gln Gly Trp Ser Asn Arg Thr Asn
                165                 170

<210>  8
<211>  172
<212>  PRT
<213>  Artificial Sequence

<220>
<223>  Codon-optimised sequence of PAD_Msp

<400>  8

Met Ser Asp Leu Phe Ala Ser Thr Arg Arg Glu Glu Ile Ala Pro Phe
1               5                   10                  15

Leu Gly Lys His Phe Ile Tyr Thr Tyr Glu Asn Gly Trp Gln Tyr Glu
            20                  25                  30

Met Tyr Ile Lys Asn Glu Arg Thr Ile Asp Tyr Arg Ile His Ser Gly
        35                  40                  45

Ile Val Gly Gly Arg Trp Val Arg Asp Gln Val Ala His Ile Val Arg
    50                  55                  60

Leu Ser Asp Glu Val Val Lys Ile Ser Trp Asp Glu Pro Thr Gly Thr
65                  70                  75                  80

Thr Val Ser Val Ala Val Asn Phe Glu Glu Arg Arg Ile His Gly Val
                85                  90                  95

Ile Phe Phe Pro Gln Trp Ile Ala Gln Asp Pro Lys Arg Thr Val Cys
            100                 105                 110

Phe Gln Asn Glu His Leu Asp Arg Met Arg Gln Tyr Arg Asp Ala Gly
        115                 120                 125

Pro Thr Tyr Pro Lys Leu Val Val Asp Glu Phe Ala Ser Val Thr Phe
    130                 135                 140

Leu Glu Asp Cys Gly Ile Asp Asn Gln Glu Val Ile Ala Cys Ala Pro
145                 150                 155                 160

Ala Asp Leu Pro Asp Gly Tyr Ala Ala Arg Gln Asn
                165                 170

## Description

### FIELD OF THE INVENTION

The present invention relates to the regio- and stereoselective hydration of a substrate compound using a decarboxylase.

### BACKGROUND OF THE INVENTION

The stereoselective addition of water across C=C double bonds is a highly valuable transformation of alkenes to nonracemic alcohols, which represents a major challenge in synthetic organic chemistry. Acid-catalysed alkene hydration -following the rule of Markovnikov — usually proceeds with low to moderate regioselectivity and is often plagued by rearrangement, which yields regioisomeric product mixtures. Despite the simplicity and the 100% atom economy of this transformation, no generally applicable protocol has been developed so far, with few examples, such as the production of *tert*-butanol from *i*-butene [1]. In a complementary fashion, base-catalysed 1,4-addition of water onto α,β-unsaturated (Michael) acceptors would be a complementary method to furnish nonracemic β-hydroxycarbonyl and -carboxyl compounds. However, due to the reversibility of 1,4-addition and the poor nucleophilicity of water, this method is not well established in asymmetric synthesis [2]. In general, the equilibrium of alkene hydration is not far from unity, it is slightly favoured in 1,4-additions, and somewhat disfavoured on isolated C=C bonds[5].

Overall, only an astonishing small number of alkene-hydration protocols are reported in literature: (i) The stereoselective hydration of α,β-unsaturated carboxylic acids using a heterobimetallic chiral biopolymer (wool-Pd^{II}-C^{II}) catalyst furnished β-hydroxy carboxylic acids in high optical purities[3], and the asymmetric *syn*-hydration of a,β-unsaturated acyl imidazoles applying a DNA-based Cu^{II} catalyst yielded β-hydroxy carbonyl compounds in moderate enantiomeric purities [4]. In order to compensate for the insufficient nucleophilicity of water, indirect methods based on various strong nucleophiles — alkoxides, *N*-silyloxycarbamates, oximes, silicon- and boron-reagents — have been employed, which require cumbersome reductive or oxidative follow-up chemistry to yield the desired β-hydroxy carbonyl or -carboxyl compounds [2].

In contrast, direct asymmetric addition of water across isolated or conjugated C=C bonds is an important process in biology [5]. The corresponding enzymes belong to the class of lyases and are termed 'hydro-lyases' or 'hydratases'. Mechanistically, these enzymes seem to fall into two categories, acting via (Lewis) acid catalysed 1,2-addition (v, vi) and (Michael-type) nucleophilic 1,4- or 1,6-addition involving quinone-methide enolates (i-iv) [6].
i) *Butenedioate hydratases* are key enzymes in the citric acid cycle and catalyse the anti-hydration of fumarate yielding (S)-malate, a process which is successfully operated in industrial scale (~2,000t/a) [7]. Unfortunately, malease, citraconase and mesaconate hydratase show a very narrow substrate spectrum [8].
ii) *Enoyl-CoA hydratases* (ECH) require the (ATP-energy-consuming) activation of their (monoacid) substrates via a thioester bond onto coenzyme A (CoA) [9, 10, 11]. ECHs, such as crotonase, are key enzymes in the β-oxidation pathway and catalyse the *syn*-hydration of *cis-* or *trans*-2-enoyl-CoA to the corresponding (R)- or (S)-3-hydroxyacyl-CoA hydration products. A few processes on industrial scale have been established, such as the hydration of crotonobetain to L-carnitine and the conversion of 2-methylpropenoic acid (obtained via enzymatic dehydrogenation of i-butyric acid) to yield (R)- or (S)-3-hydroxy-*i*-butyric acid [7]. Due to the dependence on ATP, whole cells are employed.
iii) *Hydroxycinnamoyl-CoA hydratase-lyase* (HCHL) belongs to the crotonase superfamily and requires ATP-dependent substrate activation with CoA. This enzyme catalyses the two-step degradation of feruloyl CoA via stereospecific hydration of the C=C-bond, followed by retro-aldol C-C-cleavage to yield vanillin and acetaldehyde [6, 12, 13, 14].
iv) *Michael hydratase alcohol dehydrogenase* (MhyADH) is a bifunctional enzyme, which catalyses the 1,4-hydration of a range of α,β-unsaturated carbonyl compounds followed by oxidation of the β-hydroxy moiety to yield the corresponding β-oxo-aldehyde or -ketone in the presence of an oxidant; in the absence of an electron acceptor the hydration product could be identified [15,16]. Based on sequence analysis the enzyme is a member of the molybdopterin containing oxidoreductase family comprising molybdenum, iron and zinc as cofactor. In view of the narrow substrate tolerance of hydratases, MhyADH is exceptional due to its broad substrate spectrum.
v) *Acetylene hydratase* (AH) is a rare tungsten-protein, which enables the Lewis-acid catalysed hydration of acetylene to furnish acetaldehyde [17, 18, 19, 20]. AH is a member of the dimethyl sulfoxide reductase family carrying an iron-sulfur [4Fe-4S] cluster and two molybdopterin-guanosine-dinucleotide ligands coordinated to the metal. However, the high substrate specificity and oxygen-sensitivity severely limit the practical applicability of this enzyme.
vi) The 1,2-hydration of isolated C=C bonds occurs in fatty acids (oleate hydratase yields (R)-10-hydroxy-stearate from oleic acid) [21,22], in natural products (kievitone and phaseollidin hydratase [23], carotenoid 1,2-hydratase [24]) and in terpenoids (linalool dehydratase-isomerase) [25]. The latter enzyme catalyses the hydration of myrcene to yield linalool, which is subsequently isomerised to geraniol. However, all enzymes from this group are very substrate specific.
vii) *Dehydratases* [5,8]: a number of dehydration reactions play a important role in the metabolism of carbohydrates and amino acids (galactonate and glucarate dehydratase; serine and threonine dehydratases) and since the reaction is reversible it can run in both directions (elimination versus addition of water). Diol-dehydratases act via a radical mechanism mediated by B12 as cofactor [26,27].

### DETAILED DESCRIPTION OF THE INVENTION

Herein the unprecedented stereoselective asymmetric hydration of hydroxystyrene-type substrates employing various decarboxylases is described.

One aspect of the invention relates to such process wherein the decarboxylase is selected from the group consisting of phenolic acid decarboxylase (PAD), ferulic acid decarboxylase (FDC), and β-coumaric acid decarboxylase (PDC).

A further aspect of the invention relates to a process, wherein the substrate is a compound of formula I, 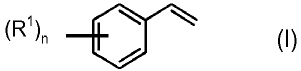 wherein
each R¹ is independently from one another selected from the group consisting of hydroxy, halogen, C₁₋₃alkoxy, C₁₋₃alkyl, C₁₋₃alkenyl, -NH₂, and
n is 0, 1, 2, 3 or 4.

A further aspect of the invention relates to a process, wherein the substrate is a compound of formula Ia, 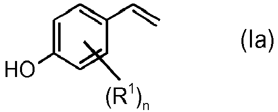 wherein
each R¹ is independently from one another selected from the group consisting of hydroxy, halogen, C₁₋₃alkoxy, C₁₋₃alkyl, C₁₋₃alkenyl, -NH₂, and n is 0, 1, 2, 3 or 4.

A further aspect of the invention relates to a process for making a compound of formula II, comprising the step of 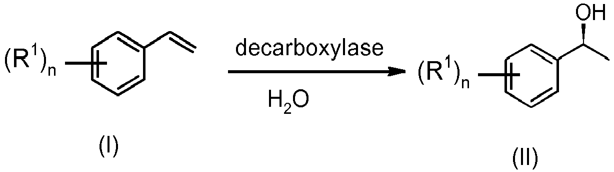 wherein
each R¹ is independently from one another selected from the group consisting of hydroxy, halogen, C₁₋₃alkoxy, C₁₋₃alkyl, C₁₋₃alkenyl, -NH₂, and n is 0, 1, 2, 3 or 4.

A further aspect of the invention relates to a process for making a compound of formula Ila, comprising the step of 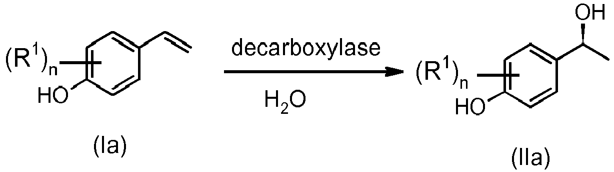 wherein
each R¹ is independently from one another selected from the group consisting of hydroxy, halogen, C₁₋₃alkoxy, C₁₋₃alkyl, C₁₋₃alkenyl, -NH₂, and n is 0, 1, 2, 3 or 4.

A further aspect of the invention relates to such process, wherein wherein a base is added.

A further aspect of the invention relates to a process, wherein the base is bicarbonate, or carbonate.

A further aspect of the invention relates to a process, wherein compound II or compound Ila is obtained with at least 30%, preferably with at least 50%, more preferred with at least 70% conversion.

A further aspect of the invention relates to such process, wherein compound II or compound Ila is obtained with at least 25%, preferably with at least 40%, more preferred with at least 55% enantiomeric excess (e.e.).

A further aspect of the invention relates to a process for manufacturing a hydrated compound, comprising the steps of:
i) suspending host cells which overexpress the decarboxylase in a buffer;
ii) adding the substrate compound;
iii) adding a base; and
iv) obtaining the hydrated compound.

A further aspect of the invention relates to a compound obtained in a process as described above for use in pharmaceutical, cosmetic and/or food industry.

The promiscuous catalytic 'hydratase-activity' of these enzymes was discovered during studies on the regioselective β-carboxylation of *p*-vinylphenol [28], which expectedly gave *p*-coumaric acid, but unexpectedly also furnished (S)-1-(*p-*hydroxyphenyl)-ethanol as side-product derived via enzymatic hydration of the C=C-bond.

Detailed literature studies revealed puzzling similarities between the (proposed) mechanism of *p*-coumaric acid decarboxylase from *Lactobacillus plantarum* [29] and that of hydroxycinnamoyl-CoA hydratase-lyase from *Pseudomonas fluorescens* [5, 6, 12], both of which act via a quinone-methide enolate intermediate. The latter represents a good Michael-acceptor for the nucleophilic 1,6-addition of water, which provides a plausible explanation for the enzymatic hydratase-promiscuity of decarboxylases.

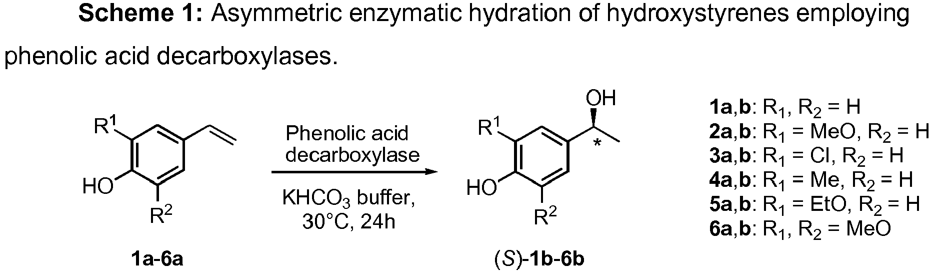

First results were obtained when the phenolic acid decarboxylases from *Lactobacillus plantarum* (PAD_Lp) and from *Bacillus amyloliquefaciens* (PAD_Ba) overexpressed in *E. coli* were applied to p-vinylphenol (**1a**) in carbonate buffer (3M, pH 8.5) to yield (S)-4-(1-hydroxyethyl)-phenol (**1b**) with good conversions (PDC_Lp: c 82%, e.e. 43%; PDC_Ba: c 64%, e.e. 53%, Table 1, entry 1). The corresponding carboxylation product (*p*-coumaric acid) was observed only as minor side product (≤ 5%). In order to examine the potential of this method, further enzymes as well as a range of various substrates were investigated. Based on similarity search (30-80% identity) using available sequences in the NCBI genebank, the following enzymes were chosen: phenolic acid decarboxylases from *Mycobacterium colombiense* (PAD_Mc), *Methylobacterium* sp. (PAD_Msp), *Pantoea* sp. (PAD_Psp), *Lactoccocuslactis* (PAD_LI), ferulic acid decarboxylase from *Enterobacter sp.* (FDC_Esp) and p-hydroxycinnamoyl CoA hydratase-lyase from *Pseudomonas fluorescens* (HCHL). All genes were synthesised at geneart AG (Regensburg) and subcloned in a common pET vector (pET 21 a or pET 28a). The obtained plasmids were transformed in a standard *E. coli* host [BL21 (DE3)] using IPTG-induction for overexpression. According to SDS-PAGE analysis, successful overexpression was obtained for all enzymes, which were employed as lyophilized whole-cell biocatalyst to a range of substrates. The absence of competing hydratase-activity of empty *E. coli* host cells was verified in separate blank experiments. To our delight, almost all decarboxylases were able to catalyse the hydration of hydroxystyrene-type substrates **1a-6a** except PDC_Mc from *Mycobacterium colombiense.* The broad range of hydratase activities was even more remarkable since the 'real' hydratase, hydroxycinnamoyl-CoA hydratase-lyase (HCHL), which was selected for reason of comparison, did not show any activity at all on the hydroxystyrene derivatives described above.

All active enzymes displayed similar levels of conversion with respect to a given substrate (c 73-82% for **1a,** c 24-45% for **2a,** c 63-76% for **3a,** c 34-47% for **4a,** c 3-25% for **5a,** c < 1 % for **6a**). Overall, the data indicate that steric effects play a major role, because *p*-vinylphenol (**1a**) and the chloro-analog **3a** were accepted best (c 63-82%, entries 1, 3) while the conversion continuously dropped by increasing the size and/or number of substituents. An additional methyl or methoxy group (**2a, 4a**), led to moderate conversions (c 24-47%, entries 2, 4), and more bulky substituents, like an ethoxy group **(5a)** resulted in a significant decrease of conversion (entry 5) indicating the limit of substrate tolerance. Compound **6a**, carrying three substituents on the phenyl ring, was not accepted as substrate (c < 1, entry 6). It seemed that electronic effects played only a minor rule.

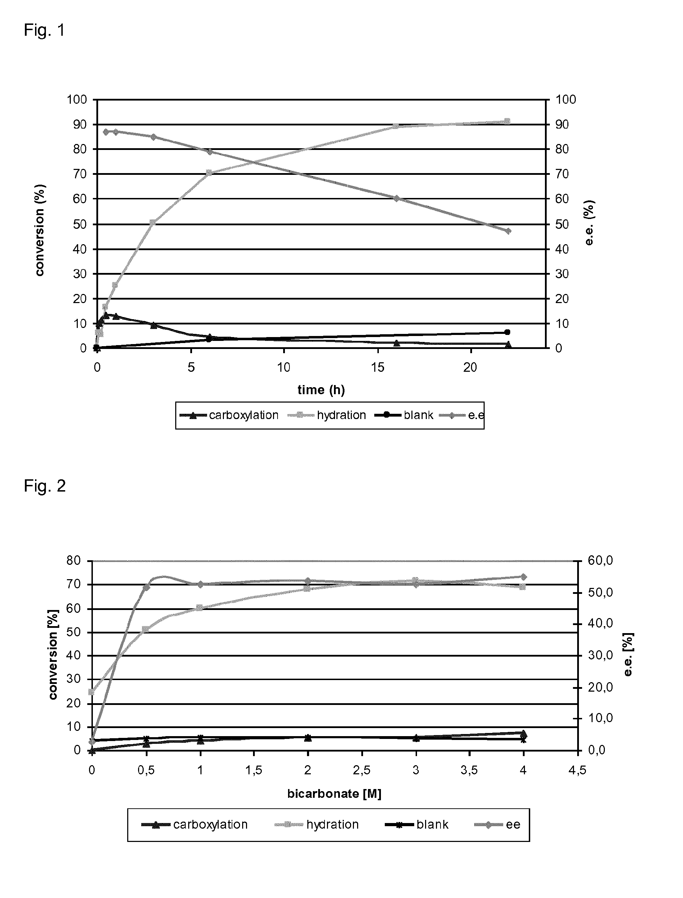

In order to get deeper insight into the enzymatic hydration, the conversion of *p-*vinylphenol (**1a**) was monitored over time employing whole cells of PAD_Lp by taking data points for e.e. and c at 0.1, 0.2, 0.5, 1, 4, 6, 16 and 22 hours (Figure 1). The conversion reached a plateau after approximately 18 hours (c 90%) and carboxylation occurred only as minor side reaction (c < 2%). However, the enantiomeric excess of hydration product **1b** continuously dropped during the course of reaction from ~ 87% at the onset of the reaction to ~ 47% at 22h reaction time. It must be mentioned that a small amount of spontaneous (non-enzymatic) background reaction took place in the absence of biocatalysts (c ~ 6%). All conversion data in Table 1 are corrected for this value accordingly to accurately describe the enzyme-catalysed activity. An approximate calculation of the stereoselectivity by excluding the non-enzymatic (spontaneous) background hydration gives an α value of ca. 20.

In order to elucidate the correlation between the hydration and the concentration of bicarbonate, a set of experiments using various buffer systems were performed using *p*-vinylphenol (**1a**) as test substrate and whole cells of PAD_Lp as biocatalyst (Figure 2). These studies showed that an increasing concentration of bicarbonate goes in hand with an increasing formation of the hydrated product **1b** reaching an optimum at 3M bicarbonate. The corresponding carboxylation product (*p*-coumaric acid) was observed only as minor side product (≤ 5%). The enantiomeric excess of the hydration product (S)-4-(1-hydroxyethyl)-phenol (1b) remained constant (e.e. ~ 55%); in the absence of bicarbonate, however, **1b** was obtained in racemic form (e.e. ≤ 1 %), which roughly correlates to the non-enzymatic (spontaneous) background reaction, which proved to be independent on the concentration of bicarbonate (c ~ 6%, blank).

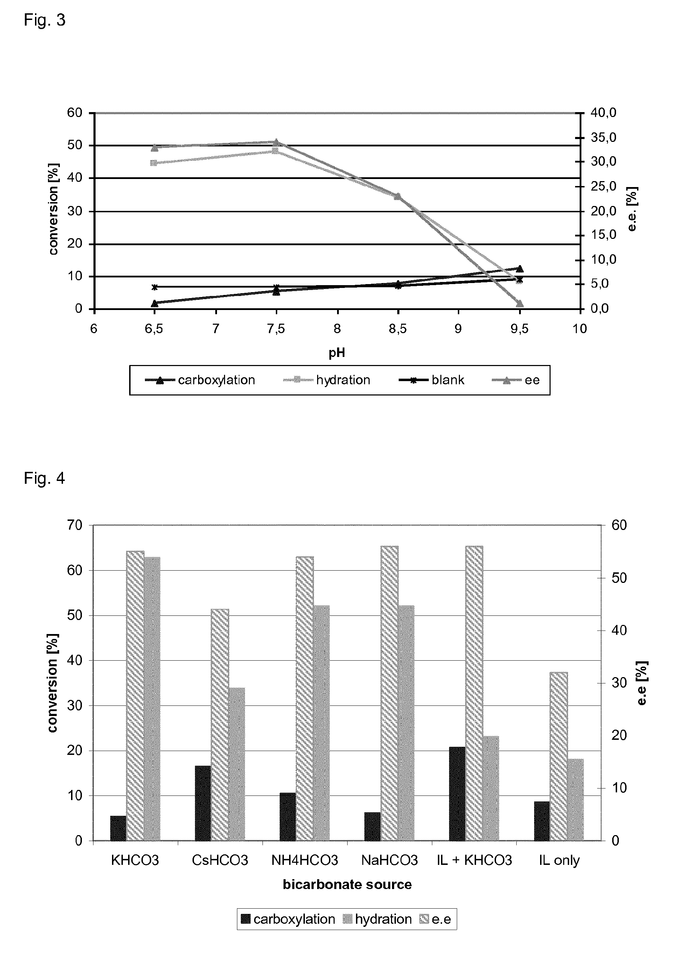

In addition, a set of experiments using carbonate buffer (1 M) at different pH values (pH 6.5, 7.5, 8.5, 9.5) (Figure 3) was performed. At pH 6.5 and 7.5 the conversion of the hydration product **1b** was around 45% and dropped significantly by increasing the pH until only spontaneous reaction at pH 9.5 (c ~8%) remained. The course of the enantiomeric excess of **1b** showed the same picture which was ~34% e.e. at pH 6.5 and 7.5 and dropped to ~23% e.e. at pH 8.5. Racemic **1b** was obtained at pH 9.5. The conversion of the carboxylation reaction increased from c ~1.5% at pH 6.5 to c ~12% at pH 9.5. Furthermore, two further buffer systems were applied: Tris-buffer (pH 8.5, 100mM) and phosphate buffer (pH 8.5, 100mM) showed hydration of 13% and 28% (e.e. ≤4%), respectively, whereas the carboxylation reaction was completely eliminated due to the absence of a CO₂ source. Overall, a significant positive influence of bicarbonate on the hydration activity was proven.

Various bicarbonate sources were applied for the hydration reaction differing in the type of the cation (Figure 4) employing *p*-vinylphenol (**1a**) as substrate and PAD_Ba as biocatalyst. Best results were obtained applying KHCO₃ (63% conversion, 55% e.e.), the concurring carboxylation reaction was only 5%. The use of NaHCO₃ displayed similar results concerning conversion and enantiomeric excess (c 52%, e.e. 56%) and the carboxylated product was obtained as minor side product (c 6%). Similar results (c 52%, e.e. 54%) were obtained with NH₄HCO₃, however, undesired carboxylation increased slightly (c 10%). The use of CsHCO₃ resulted in a significant decrease of conversion and enantiomeric excess in the hydration (c 34%, e.e. 44%) and the ratio between hydration and carboxylation became unfavorable, which was even worse using carbonate-based ionic liquids (1-butyl-3-methylimidazolium hydrogen carbonate solution ~50% in methanol/water 2/3; 200 µL).

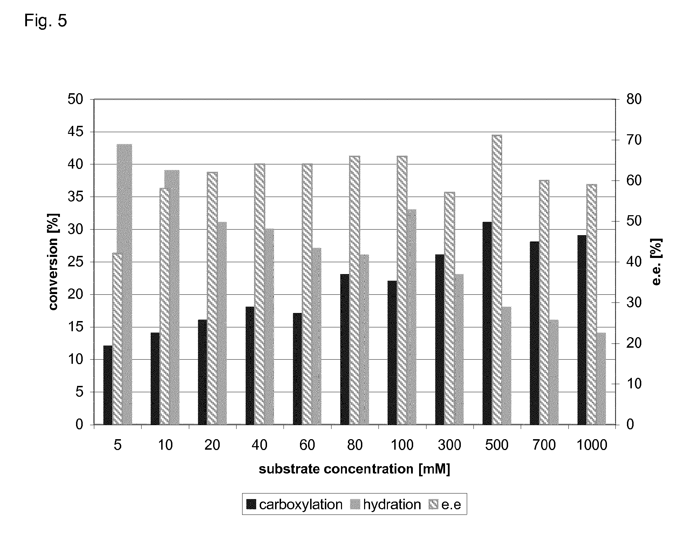

The influence of the ratio of hydration versus carboxylation depending on the substrate concentration was investigated by using 2-methoxy-4-vinylphenol (**2a**) and PaD_Ba. Figure 5 shows that the hydration clearly dominates over the carboxylation at reduced substrate concentrations, while the e.e. of **2b** was not significantly affected.

**Table 1. Activities and stereoselectivities of the asymmetric enzymatic hydration of hydroxystyrenes 1a-6a.**

| **Entry** | **Substrate** | **Enzyme Product** | | **PAD_Lp** | **PAD_ Ba** | **PAD_Mc** | **PAD_Ms** | **PAD_Ps** | **PAD_L1** | **FDC_Es** | **blank^{a}** |
|---|---|---|---|---|---|---|---|---|---|---|---|
| **1** | 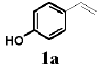 | 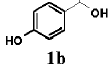 | c [%] | 82 | 64 | 2 | 77 | 75 | 73 | 77 | 6 |
| | | | e e [%] | 43 (*S*) | 53 (*S*) | n d | 40 (*S*) | 36 (*S*) | 25(*S*) | 41 (*S*) | *rac* |
| **2** | 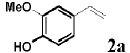 | 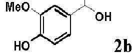 | c [%] | < 1 | 30 | < 1 | 24 | 39 | 45 | 27 | 14 |
| | | | e e [%] | - | 50 (*S*) | - | 28 (*S*) | 12 (*S*) | 28 (*S*) | 8 (*S*) | *rac* |
| **3** | 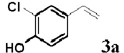 | 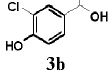 | c [%] | 73 | 73 | < 1 | 63 | 76 | 73 | 74 | 17 |
| | | | e e [%] | 28 (*S*) | 36 (*S*) | - | 27 (*S*) | 10 (*S*) | 20 (*S*) | 8(*S*) | *rac* |
| **4** | 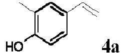 | 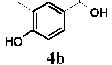 | c [%] | 46 | 46 | < 1 | 45 | 44 | 47 | 34 | 39 |
| | | | e e [%] | 29 (*S*) | 44 (*S*) | - | 37 (*S*) | 32 (*S*) | 18 (*S*) | 71 (*S*) | *rac* |
| **5** | 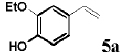 | 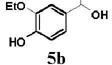 | c [%] | 8 | 3 | < 1 | 5 | 12 | 35 | 17 | 10 |
| | | | e e [%] | n d | n d | - | n d | 3^{b} | 8^{b} | 10^{b} | *rac* |
| **6** | 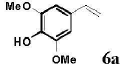 | 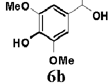 | c [%] | <1 | <1 | <1 | <1 | <1 | <1 | <1 | <1 |
| | | | e e [%] | - | - | - | - | - | - | - | - |

Reaction conditions: whole lyophilised cells containing the overexpressed enzyme (30mg), substrate (10mM), KHCO₃ (3M), Pi buffer (pH 5.5, 100mM), final pH ~ 8.5, 30°C, 120rpm, 24h; n.d. = not determined due to low conversion;^{a} spontaneous (non-enzymatic) hydration in absence of biocatalyst; ^{b} absolute configuration not determined due to low e.e.

### DESCRIPTION OF THE DRAWINGS

Figure 1: Time study of the hydration of *p*-vinylphenol (**1a**) employing PAD_Lp.; e.e. of **1b**.
Figure 2: Hydration of *p*-vinylphenol (**1a**) employing PAD_Lp using various concentrations of bicarbonate; e.e. of **1b**.
Figure 3: Hydration of *p*-vinylphenol (**1a**) employing PAD_Lp using a carbonate buffer (1M) at different pH values; e.e. of **1b**.
Figure 4: Hydration of *p*-vinylphenol (**1a**) employing PAD_Ba using various bicarbonate salts; e.e. of **1b**; IL = ionic liquid (1-butyl-3-methylimidazolium hydrogen carbonate solution ~50% in methanol/water 2/3; 200µL).
Figure 5: Relative ratio of hydration versus carboxylation depending on the substrate concentration employing 2-methoxy-4-vinylphenol (**2a**) and PaD_Ba; e.e. of **2b**.

The subject matter of the following definitions is considered embodiments of the present invention:
1. A process for the stereoselective hydration of a substrate compound using a decarboxylase.
2. The process according to claim 1, wherein the decarboxylase is selected from the group consisting of phenolic acid decarboxylase (PAD), ferulic acid decarboxylase (FDC), and β-coumaric acid decarboxylase (PDC).
3. The process according to claim 1 or 2, wherein the substrate is a compound of formula 1, 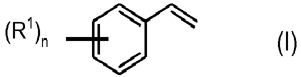 wherein
   each R¹ is independently from one another selected from the group consisting of hydroxy, halogen, C₁₋₃alkoxy, C₁₋₃alkyl, C₁₋₃alkenyl, -NH₂, and
   n is 0, 1, 2, 3 or 4.
4. The process according to claim 1 or 2, wherein the substrate is a compound of formula Ia, 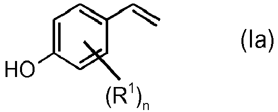 wherein
   each R¹ is independently from one another selected from the group consisting of hydroxy, halogen, C₁₋₃alkoxy, C₁₋₃alkyl, C₁₋₃alkenyl, -NH₂, and
   n is 0, 1, 2, 3 or 4.
5. A process for making a compound of formula II, comprising the step of 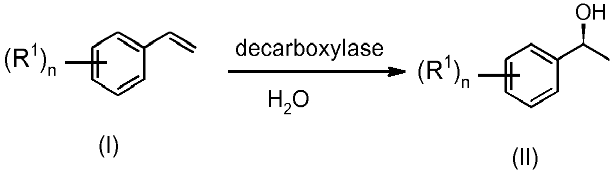 wherein
   each R¹ is independently from one another selected from the group consisting of hydroxy, halogen, C₁₋₃alkoxy, C₁₋₃alkyl, C₁₋₃alkenyl, -NH₂, and
   n is 0, 1, 2, 3 or 4.
6. A process for making a compound of formula Ila, comprising the step of 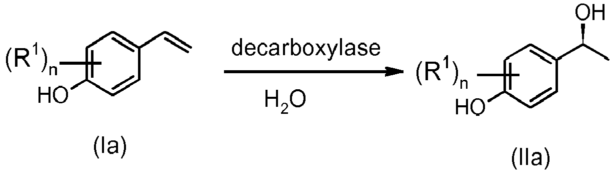 wherein
   each R¹ is independently from one another selected from the group consisting of hydroxy, halogen, C₁₋₃alkoxy, C₁₋₃alkyl, C₁₋₃alkenyl, -NH₂, and
   n is 0, 1, 2, 3 or 4.
7. The process according to claim 5 or 6, wherein a base is added.
8. The process according to claim 5 or 6, wherein the base is bicarbonate, or carbonate.
9. The process according to any one of claims 5 to 8, wherein compound II or compound Ila is obtained with at least 30%, preferably with at least 50%, more preferred with at least 70% conversion.
10. The process according to any one of claims 5 to 9, wherein compound II or compound Ila is obtained with at least 25%, preferably with at least 40%, more preferred with at least 55% enantiomeric excess (e.e.).
11. A process for manufacturing a hydrated compound, comprising the steps of:
   i) suspending host cells which overexpress the decarboxylase in a buffer;
   ii) adding the substrate compound;
   iii) adding a base; and
   iv) obtaining the hydrated compound.
12. A compound obtained in a process according to claim 11 for use in pharmaceutical, cosmetic and/or food industry.

### Examples

### Materials and Methods

2-Methoxy-4-vinylphenol (**2a**), KHCO₃, KH₂PO₄ and Na₂PO₄*2 H₂O were from Sigma Aldrich, p-vinylphenol (**1a**) was purchased from Alfa Aesar, petroleum ether (boiling range 60 - 95°C) was acquired from VWR.

TLCs were run on silica plates (Merck, silica gel 60, F₂₅₄); for column chromatography silica gel (Merck, silica gel 60) was used, compounds were detected using UV (254nm); NMR experiments were acquired either on a Bruker Avance III 300MHz spectrometer using a 5 mm BBO probe with z-axis gradients at 300 K. Chemical shifts (δ) are reported in ppm and coupling constants (J) are given in Hz. All GC-MS measurements were carried out on an Agilent 7890A GC system, equipped with an Agilent 5975C mass selective detector (electron impact, 70 eV) and a HP-5-MS column (30 m x 0.25 mm x 0.25µm) using helium as carrier gas at a flow of 0.55 mL/min. Following temperature program was used in all GC-MS measurements: initial temperature: 100°C, hold for 0.5 min, 10°C/min, to 300°C.

Phenolic acid decarboxylase from *Lactobacillus plantarum* (PAD_Lp, GI: 300769086) and from *Bacillus amyloliquefaciens* (PAD_Ba, GI: 308175189), subcloned in a pET 28a (+) vector, were provided by Byung-Gee Kim (Seoul National University, Korea). Phenolic acid decarboxylase from *Mycobacterium colombiense* (PAD_Mc), *Methylobacterium* sp. (PAD_Msp), *Pantoea* sp. (PAD_Psp), *Lactoccocuslactis* (PAD_LI), ferulic acid decarboxylase from *Enterobacter* sp. (FDC_Esp) and p-hydroxycinnamoyl CoA hydratase-lyase from *Pseudomonas fluorescens* (HCHL) were synthesized at Geneart AG (Germany, Regensburg). Their DNA sequences were allocated in the NCBI Genebank (GI: 3116017 for HCHL, GI: 323462934 for FDC_Esp, GI: 304396594 for PAD_Psp, GI: 15673912 for PAD_LI, GI: 342860341 for PAD_Mc, GI: 168197631 for PAD_Msp).

Codon-optimised sequences of decarboxylases and HCHL (SEQ ID NO:1) HCHL (GI: 3116017)

MSTYEGRWKTVKVEIEDGIAFVILNRPEKRNAMSPTLNREMIDVLETLEQDPAAGVLV
LTGAGEAWTAGMDLKEYFREVDAGPEILQEKIRREASQWQWKLLRMYAKPTIAMVN
GWCFGGGFSPLVACDLAICADEATFGLSEINWGIPPGNLVSKAMADTVGHRQSLYYI
MTGKTFGGQKAAEMGLVNESVPLAQLREVTIELARNLLEKNPVVLRAAKHGFKRCRE
LTWEQNEDYLYAKLDQSRLLDTEGGREQGMKQFLDDKSIKPGLQAYKR

PAD_Ba (GI: 308175189) (SEQ ID NO:2)

MENFIGSHMIYTYENGWEYEIYIKNDHTIDYRIHSGMVGGRWVRDQEVNIVKLT
EGVYKVSWTEPTGTDVSLNFMPNEKRMHGIIFFPKWVHEHPEITVCYQNDYIDVMKE
SREKYDTYPKYVVPEFADITYLNNAGINNEALISEAPYEGMTDDIRAGKLK

PAD_Lp (GI: 300769086) (SEQ ID NO:3)

MTKTFKTLDDFLGTHFIYTYDNGWEYEWYAKNDHTVDYRIHGGMVAGRWVT
DQKADIVMLTEGIYKISWTEPTGTDVALDFMPNEKKLHGTIFFPKWVEEHPEITVTYQN
EHIDLMEQSREKYATYPKLVVPEFANITYMGDAGQNNEDVISEAPYKEMPNDIRNGKY
FDQNYHRLNK

FDC_Esp (GI: 323462934) (SEQ ID NO:4)

MNTFDKHDLSGFVGKHLVYTYDNGWNYEIYVKNDNTIDYRIHSGLVGNRWVK
DQEAYIVRVGESIYKISWTEPTGTDVSLIVNLGDSLFHGTIFFPRWVMNNPEPTVCFQ
NDHIPLMNSYREAGPAYPTEVIDEFATITFVRDCGANNESVIACAASELPKNFPDNLK

PAD_Psp (GI: 304396594) (SEQ ID NO:5)

MSTFDKHDLSGIVGKHLVYTYDNGWNYELYIKNASTIDYRIHSGMVGNRWVKN
QHVYVVRLAQDVYKVSWTEPTGTDVSLAVNLADKIFHGTIFFPRWVMNNPEKTVCFQ
NDHLEEMAKFRETGPAYPTEIIDEFATLTVIREVGENNESVIDCPPDQLPANWPANLQ
N

PAD_LI (GI: 15673912) (SEQ ID NO:6)

MKTFKSLEDFVGTHFIYTYDNGWEYELYVKNDHTIDYRIHGGMVAGRWVKDQ
EVSLVMLTEGIYKITWTEPTGTDVALDFLPNEGKLHGMIFFPKWVEEHPEITVCFQND
FIDLMHESREKYETYPKYLVPEFAKITYAAEAGKDNDDVVAQAPYKEMTNDIRNGKYF
DQNYKMIKH

PAD_Mc (GI: 342860341) (SEQ ID NO:7)

MTSVTNPIPPQDLSGIVGHRFIYTYANGWQYEMYVKNVTTIDYRIHSGHVGGR
WVKGQQVNLVQLDDDSFKISWTEPTGTCVAVNVLPGKRRIHGVIFFPQWIRMHGEHT
VCFQNDHLDEMRAYRDRGPTYPIYEVPEFAYITLFEYVGTDDETVIDTGPEHLPQGW
SNRTN

PAD_Msp (GI: 168197631) (SEQ ID NO:8)

MSDLFASTRREEIAPFLGKHFIYTYENGWQYEMYIKNERTIDYRIHSGIVGGRW
VRDQVAHIVRLSDEVVKISWDEPTGTTVSVAVNFEERRIHGVIFFPQWIAQDPKRTVC
FQNEHLDRMRQYRDAGPTYPKLVVDEFASVTFLEDCGIDNQEVIACAPADLPDGYAA
RQN

### Cloning and overexpression of enzymes

Genes were synthesized at Geneart AG (Germany, Regensburg) and subcloned in a pET vector (pET21 a or pET28a). The obtained plasmid was then transformed into chemically competent *Escherichia coli* BL21 (DE3) cells and heterologous overexpression was performed as follows:

For preculturing 500mL LB medium [Trypton (10g/L, Oxoid L0042), yeast extract (5g/L, Oxoid L21), NaCl (5g/L, Roth 9265.1)] supplemented with the appropriate antibiotics [ampicillin (100µg/mL, Sigma Aldrich) for PAD_Mc, PAD_Msp, PAD_Psp, PAD_LI and FDC_Esp], kanamycin (50µg/mL, Roth) for HCHL, PAD_Lp and PAD_Ba] were inoculated with 3mL ONC (starter culture) and incubated at 37°C and 120 rpm until an OD₆₀₀ of 0.6-1.0 was reached. Then IPTG [175 µg/mL, 0.5 mM, Peqlab] was added for induction and the cells left overnight at 20°C and 120 rpm. The next day the cells were harvested by centrifugation (20 min, 8000 rpm, 4°C), washed with phosphate buffer (5mL, 50mM, pH 7.5) and centrifuged under the same conditions. The cell pellet thus obtained was resuspended in phosphate buffer (5 mL, 50 mM, pH 7.5), shock frozen in liquid nitrogen followed by lyophilization. Lyophilized cells were stored at +4°C.

### Example 1: General procedure for the asymmetric enzymatic hydration of substrates 1a-6a

Enzymatic hydration reactions were performed in glass vials capped with septums. Lyophilized whole cells (30mg *E. coli* host cells containing the corresponding overexpressed enzyme) were suspended in phosphate buffer (1 mL, pH 5.5, 100 mM) and were rehydrated for 30 min. The substrate (10 mM) was added, followed by addition of KHCO₃ (3M, 300 mg). Thereafter the vials were immediately tightly closed and the mixture was shaken at 30°C and 120 rpm. After 24h the mixture was centrifuged (13000 rpm, 15 min), an aliquot of 100 µL of each sample was diluted in 1 mL of H₂O/MeCN (1:1) supplemented with TFA (30 µL). After incubation at room temperature for 5 min, the samples were again centrifuged (13000 rpm, 15 min) and analyzed on reverse-phase HPLC to determine the conversion.

For the determination of the enantiomeric excess, products were extracted after 24h with EtOAc (2 x 500 µL) and the combined organic phases were dried over Na₂SO₄. The organic solvent was removed, the residue was dissolved in n-heptane/i-propanol (90/10) and analyzed on HPLC to determine the e.e.

### Determination of absolute configuration

The absolute configuration of hydration products **2b-5b** was determined via coinjection of the hydration product with independently synthesised reference material of known absolute configuration. The latter were obtained via oxidative kinetic resolution of *rac*-alcohols or by asymmetric bioreduction of the corresponding ketones using alcohol dehydrogenases of known stereopreference: (S)-selective ADH-A from *Rhodococcus ruber* [1,2], (S)-selective ADH-005 (corresponds to ADH-T from Jülich chiral solutions) and (R)-selective ADH from *Lactobacillus brevis* [3]. Additional proof was obtained by comparison of optical rotation values for **1b** and **2b** with literature data.

### Example 2: 4-(1-Hydroxyethyl)-2-methoxyphenol (2b)

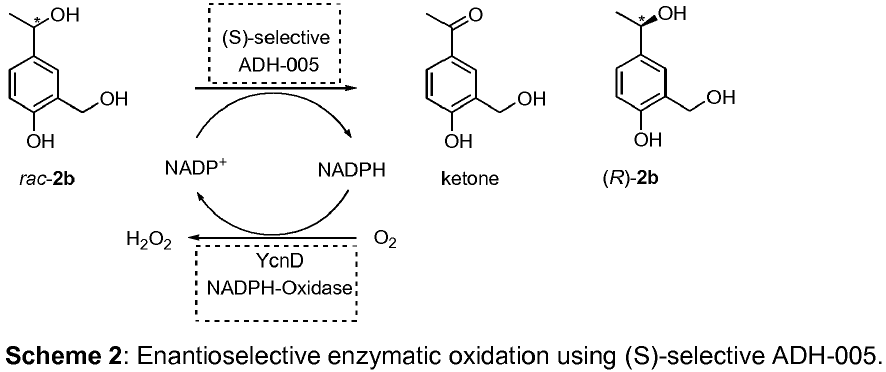

An aliquot of ADH-005 (50 µL, 77.5U, corresponds to ADH-T, Jülich chiral solutions) was added to a phosphate buffer solution (950µL, 50mM, pH 7.5) containing *rac*-4-(1-hydroxyethyl)-2-methoxyphenol (50 mM), cofactor NADP⁺ (1 mM) and nicotinamide oxidase YncD (10 µL) for cofactor-recycling [4, 5]. The mixture was incubated at 30°C and 120 rpm. After 24h products were extracted with EtOAc (2x500 µL). The combined organic phases were dried over Na₂SO₄, the organic solvent was removed and the residue was dissolved in a mixture of *n*-heptane/i-propanol (90:10). The samples were analyzed on a chiral HPLC column to determine the enantiomeric excess.

### Example 3: 4-(1-Hydroxyethyl)phenol (1 b), 4-(1-hydroxyethyl)-2-chlorophenol (3b) and 4-(1-hydroxyethyl)-2-methylphenol (4b)

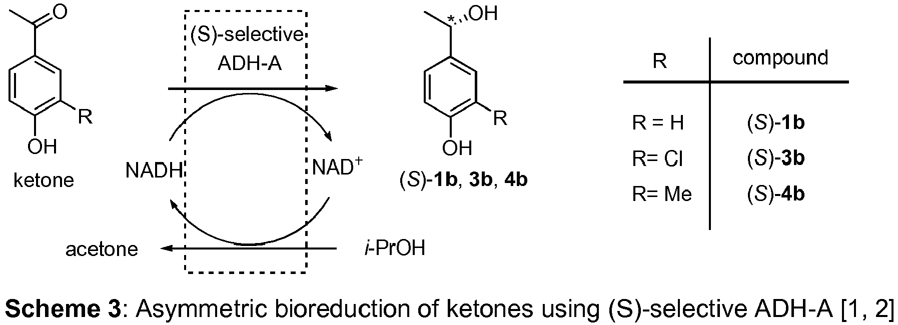

An aliquot of ADH-A (50µL, 3U) was added to a phosphate buffer solution (950 µL, 50 mM, pH 7.5) containing the substrate (4-hydroxyacetophenone, 3-chloro-4-hydroxyacetophenone, or 3-methyl-4-hydroxyaceophenone, 50mM), the cofactor NAD⁺ (1 mM) and the cosubstrate *i*-propanol (5 µL). The mixture was incubated at 30°C and 120 rpm. After 24h products were extracted with EtOAc (2x500 µL). The combined organic phases were dried over Na₂SO₄, the solvent was removed and the residue was dissolved in *n*-heptane/*i*-propanol (90:10). The samples were analyzed on a chiral HPLC to determine the enantiomeric excess.

### Example 4: Determination of optical rotation of (S)-4-(1-hydroxy-ethyl)phenol (1 b) and (S)-4-(1-hydroxyethyl)-2-methoxy-phenol (2b)

To determine the optical rotation value of 4-(1-hydroxyethyl)phenol (**1b**) and 4-(1-hydroxyethyl)-2-methoxyphenol (**2b**) the asymmetric hydration of **1a** and **2a** applying PAD from *Bacillus amyloliquefaciens* was scaled up 10 times. After 24h, hydration products were extracted with EtOAc (2 x 500 µL) and the combined organic phases were dried over Na₂SO₄. Afterwards the solvent was removed and the optical rotation was measured at 589 nm (Na-line) using a Perkin-Elmer polarimeter 341.

**4-(1-Hydroxyethyl)phenol (1b)**: [α]_{D}²⁰ -37 (c 0.125, EtOH). The absolute configuration of **1b** was determined to be (S) by comparison with literature values [α]_{D}²⁰-48 (c 4.2, EtOH) [6].

**4-(1-Hydroxyethyl)-2-methoxyphenol (2b)**: [α]_{D}²⁰-41 (c 0.23, CHCl₃). The absolute configuration of 2b was determined to be (S) by comparison with literature values [α]_{D}²⁰+41 (c 0.8, CHCl₃) for the (R)-enantiomer [7].

### Example 5: Synthesis of substrates

Hydroxystyrenes **3a-6a** were synthesized according to the following general Wittig-reaction protocol [8]

**2-Chloro-4-vinylphenol (3a), 2-methyl-4-vinylphenol (4a), 2-ethoxy-4-vinylphenol (5a) and 2,6-dimethoxy-4-vinylphenol (6a): Sodium** bis(trimethylsilyl)amide (1.05 g, 5.7 mmol, Sigma Aldrich) was added under cooling to a stirred solution of methyl triphenylphosphonium bromide (1.85g, 5.2mmol, Sigma Aldrich) in freshly distilled THF (8 mL). Immediately afterwards a yellow colour change could be observed. After 1.5h of stirring the corresponding solid aldehyde (2.54 mmol, Sigma Aldrich) was added to the ylid-solution and the stirring was continued for 4h. The mixture was acidified using H₂SO₄ (0.1 M, 5 mL) and extracted with CH₂Cl₂. The combined organic phases were dried over Na₂SO₄, evaporated and purified by flash chromatography on silica.

**2-Chloro-4-vinylphenol (3a)**: Eluent for flash chromatography: petroleum ether/EtOAc (2:1), isolated yield 80% (0.31 g, 2.03 mmol), TLC: R_{f} = 0.63 (silica, petroleum ether/EtOAc 2:1); GC-MS: m/z 154; ¹H-NMR (MeCN-d₃): δ 5.07 (1H, dd, J = 0.56 and 10.95); δ 5.58 (1H, dd, J = 0.67 and 17.63); δ 6.54 (1H, dd, J = 10.95 and 17.63); δ 6.84 (1H, d, J = 8.40); δ 7.17 (1H, dd, J = 1.97 and 8.21); δ 7.35 (1H, d, J = 2.03); ¹³C-NMR (MeCCN-d₃):δ 112.3, 116.6, 120.2, 126.0, 127.4, 131.1, 135.1, 152.0.

**2-Methyl-4-vinylphenol (4a)**: Eluent for flash chromatography: petroleum ether/EtOAc (5:1), isolated yield 63% (0.21 g, 1.60 mmol), TLC: R_{f} = 0.48 (silica, petroleum ether/EtOAc 5:1); GC-MS: m/z 134; ¹H-NMR (MeCN-d₃): δ 2.07 (3H, s), δ 4.96 (1H, dd, J = 0.79 and 10.93); δ 5.50 (1H, dd, J = 0.87 and 17.64); δ 6.53 (1H, dd, J = 10.94 and 17.65); δ 6.64 (1H, d, J = 8.22); δ 6.76 (1H, s); δ 7.02 (1H, dd, J = 1.66 and 8.21); δ 7.12 (1H,s); ¹³C-NMR (MeCN-d₃): δ 15.14, 110.3, 114.7, 124.4, 124.9, 128.7, 129.6, 136.6, 154.9.

**2-Ethoxy-4-vinylphenol (5a)**: Eluent for flash chromatography: petroleum ether/EtOAc (10:1), isolated yield 16% (67.3 mg, 0.41 mmol), TLC: R_{f} = 0.39 (silica, petroleum ether/EtOAc 10:1); GC-MS: m/z 164 ¹H-NMR (acetone-d₆): δ 1.39 (3H, t, J = 6.98); δ 4.13 (2H, q, J = 6.98); δ 5.05 (1H, dd, J = 0.78 and 10.91); δ 5.62 (1H, dd, J = 0.89 and 17.60); δ 6.65 (1H, dd, J = 10.91 and 17.61); δ 6.80 (1H, d, J = 8.13); δ 6.91 (1H, dd, J = 1.75 and 8.13), δ 7.09 (1H, d, J = 1.73), δ 7.65 (1H, s); ¹³C-NMR (acetone-d₆): δ 14.17, 64.12,109.93, 110.14, 114.82, 119.72, 129.69, 136.93, 146.72, 146.93. (acetone-d₆ contained H₂O δ H₂O peak in NMR)

**2,6-Dimethoxy-4-vinylphenol (6a)**: Eluent for flash chromatography: CH₂Cl₂/MeOH (10:1), isolated yield 59% (0.27 g, 1.50 mmol), TLC: R_{f} = 0.94 (silica, CH₂Cl₂/MeOH 10:1); GC-MS: m/z 180; ¹H-NMR (CDCl): δ 3.93 (6H, s); δ 5.17 (1H, dd, J = 0.61 and 10.83); δ 5.56 (1H, s); δ 5.62 (1H, dd, J = 0.70 and 17.50); δ 6.64 (1H, dd, J = 10.78 and 17.47); δ 6.67 (2H, s); ¹³C-NMR (CDCl₃): δ 56.24, 56.27, 103.0, 111.8, 129.2, 134.8, 136.8, 147.1. NMR data corresponded to literature [9].

### Example 6: Synthesis of reference materials

Racemic reference material for alcohols **1b**, **3b** and **4b** was obtained via reduction of the corresponding ketones.

***rac*-4-(1-Hydroxyethyl)phenol (1b), rac-4-(1-hydroxyethyl)-2-chlorophenol (3b) and *rac*-4-(1-hydroxyethyl)-2-methylphenol (4b)** [10]: Ketone (6 mmol, Sigma Aldrich) was added to a stirred solution of CeCl₃ x 7H₂O (6 mmol, Sigma Aldrich) in methanol (20 mL). Then sodium borohydride (6 mmol) was added in small portions over 3 min, the once clear solution turned milky-white and the stirring was continued for 20 min at room temperature. The mixture was quenched with a saturated (NH₄)₂SO₄-solution (50 mL) and extracted with diethyl ether (3 x 30 mL). The combined organic phases were washed with brine, dried over Na₂SO₄, evaporated and purified by flash chromatography on silica.

**4-(1-Hydroxyethyl)phenol (1b)**: Eluent for flash chromatography: petroleum ether/EtOAc (1:2), isolated yield 79% (0.72 g, 5.24 mmol), TLC: R_{f} = 0.38 (silica, petroleum ether/EtOAc 1:2); GC-MS: m/z 138. ¹H-NMR (MeCN-d₃): δ 1.37 (3H, d, J = 6.43), δ 3.14 (1H, d, J = 3.94); δ 4.70-4.78 (1H, m); δ 6.78 (2H, d, J = 8.54); δ 6.89 (1H,s); δ 7.21 (2H, d, J = 8.41 ); ¹³C-NMR (MeCN-d₃): δ 24.83, 68.81, 114.8, 126.7, 138.1, 155.8.

**4-(1-Hydroxyethyl)-2-chlorophenol (3b)**: Eluent for flash chromatography: petroleum ether/EtOAc (1:2), isolated yield 78% (0.82 g, 4.76 mmol), TLC: R_{f} = 0.70 (silica, petroleum ether/EtOAc 1:2); GC-MS: m/z 172. ¹H-NMR (MeCN-d₃): δ 1.36 (3H, d, J = 6.44), δ 3.25 (1H,s), δ 4.43 (1H, q, J = 6.16 and 6.20); δ 6.93 (1H, d, J = 8.34); δ 7.16 (1H, dd, J = 1.93 and 8.39; δ 7.34 (1H, d, J = 2.01); ¹³C-NMR (MeCN-d₃): δ 24.74, 68.22, 116.3, 119.6, 125.3, 126.8, 140.0, 151.1.

**4-(1-Hydroxyethyl)-2-methylphenol (4b)**: Eluent for flash chromatography: petroleum ether/EtOAc (1:1), isolated yield 87% (0.70 mg, 4.58 mmol), TLC: R_{f} = 0.51 (silica, petroleum ether/EtOAc 1:1); GC-MS: m/z 152. ¹H-NMR (MeOH-d₄): δ 1.29 (3H, d, J = 0.47), δ 2.07 (1H, s); δ 4.59 (1H, q, J = 6.44); δ 6.58 (1H, d, J = 8.17); δ 6.88 (1H, dd, J = 8.17 and 2.07); δ 6.89 (1H, s) (H₂O peak from solvent: δ 4.78, 2H, s); ¹³C-NMR (MeOH-d₄): δ 16.45, 25.58, 70.84, 115.4, 125.1, 125.4, 129.3, 138.3, 155.8.

**4-(1-Hydroxyethyl)-2-ethoxyphenol (5b)**: 3-ethoxy-4-hydroxybenzaldehyde (1.9mmol, 310 mg, Sigma Aldrich) dissolved in anhydrous diethyl ether (2 mL) was added under Argon to a stirred solution of Cs₂CO₃ (1.95g, 6 mmol, Sigma Aldrich) in diethylether (4 mL) at room temperature. After 30 min methyl magnesium bromide (1 mL of a 3M solution, 3 mmol, Sigma Aldrich) was slowly added under ice-cooling and stirring was continued for 2h. Thereafter diethylether (1 mL) and another portion of methyl magnesium bromide (1 mL) were added and stirring was continued overnight. The reaction was quenched with a saturated NH₄Cl-solution, the pH was set to 4 with HCl (6M) and products were extracted with diethyl ether (3 x 30 mL). The combined organic phases were dried over Na₂SO₄, the solvent was evaporated and products were purified by flash chromatography on silica using petroleum ether/EtOAc (1:1) as eluent; isolated yield 6.4% (22 mg, 0.12 mmol), TLC: R_{f} = 0.51 (silica, petroleum ether/EtOAc 1:1); GC-MS: m/z 182. ¹H-NMR (MeOH-d₄₎: δ 1.40-1.45 (6H, m), δ 4.10 (2H, q, J = 6.99); δ 4.74 (1H, q, J = 6.42); δ 6.75-6.81 (2H, m); δ 6.96 (1H, s) (H₂O peak from solvent: δ 4.89, 2H, s) ; ¹³C-NMR (MeOH-d₄): δ 13.78, 24.16, 64.10, 69.39, 110.23, 114.56, 117.85, 137.72, 145.50, 146.54.

### Example 7: Determination of conversion

All analyses were carried out on a Shimadzu HPLC system from equipped with a diode array detector (SPD-M20A) and a reversed-phase Phenomenex Luna column C18 (2) 100A 250*4.600 mm 5 µm, column temperature 24°C. Conversions were determined by comparison with calibration curves for products and substrates prepared with authentic reference material.

The method for **1a/1b**, **2a/2b** and **5a/5b** was run over 12 min with H₂O/TFA (0.1 %) as the mobile phase at a flow rate of 1 mL/min and a MeCN/TFA (0.1 %) gradient (0-2 min 15%, 2-10 min 0-100%, 10-12 min 100%). The column temperature was 24°C and compounds were spectrophotometrically detected at 254 or 270 nm. Retention times: **1a** 10.83 min, 1b 7.62 min, **2a** 11.01 min, **2b** 7.90 min, **5a** 11.81 min and **5b** 8.77 min.

The method for **3a/3b** and **4a/4b** was run over 17 min with H₂O/TFA (0.1 %) as the mobile phase at a flow rate of 1 mL/min and a MeCN/TFA (0.1 %) gradient (0-2 min 0%, 2-15 min 0-100%, 15-17 min 100%). The column temperature was 24°C and compounds were spectrophotometrically detected at 270 and 280 nm respectively. Retention times: 3a 15.22 min, 3b 11.75 min 4a 15.11 min, 4b 11.24 min.

The method for 6a was run over 32 min with H₂O/TFA (0.1 %) as the mobile phase at a flow rate of 0.5 mL/min and an MeCN/TFA (0.1 %) gradient (0-2 min 0%, 2-30 min 0-100%, 30-32 min 100%). Compounds were spectrophotometrically detected at 280 nm. Retention time: 6a 26.20 min.

### Example 8: Determination of enantiomeric excess

All analyses were carried out on a normal-phase Shimadzu HPLC system. The absolute configurations were determined by coinjection with independently synthesised reference material, which was obrained as described above.

The enantiomeric excess of **1b-5b** was determined using a Chiralcel OD-H column (25 cm x 0.46 cm). The method for **1b**, **2b**, **4b** and **5b** was run over 20 min with n-heptane/i-propanol (90:10) as the mobile phase at a flow rate of 1.0 mL/min. The column temperature was 25°C and compounds were spectrophotometrically detected at 270 nm or 280 nm. Retention times: (R)-**1b** 11.40 min, (S)-**1b** 12.40 min, (R)-**2b** 15.26 min, (S)-**2b** 16.26 min, (R)-**4b** 9.30 min, (S)-**4b** 10.79 min, rac-**5b** 11.28 and 12.51 min, absolute configuration was not determined.

The method for **3b** was run over 60 min with n-heptane/i-propanol (95:5) as the mobile phase at a flow rate of 0.5 mL/min. The column temperature was 25°C and compounds were spectrophotometrically detected at 280 nm. Retention times: (R)-**3b** 39.66 min, (S)-**3b** 43.17 min.

### References:

[1] H.-D. Hahn, G. Dämbkes, N. Rupprich, H. Bahl, Butanols in: Ullmann's Encyclopedia of Industrial Chemistry, Electronic version, Wiley-VCH, Weinheim, 2010.
[2] E. Hartmann, D. J. Vyas, M. Oesterreich, Chem. Commun. 2011, 47, 7917-1932.
[3] S.-Q. Wang, Z.-W. Wang, L.-C. Yang, J.-I. Dong, C.-Q. Chi, D.-N. Sui, Y.-Z. Wang, J.-G. Ren, M.-Y. Hung, Y.-Y. Jiang, J. Mol. Catal. A: Chem. 2007, 264, 60-65.
[4] A. J. Boersma, D. Coquiere, D. Geerdink, F. Rosati, B. L. Feringa, G. Roelfes, Nat. Chem. 2010, 2, 991-995.
[5] J. Jin, U. Hanefeld, Chem. Commun. 2011, 47, 2502-2510.
[6] J. P. Bennett, L. Bertin, B. Moulton, I. J. S. Fairlamb, A. M. Brzozowski, N. J. Walton, G. Grogan, Biochem. J. 2008, 414, 281-289.
[7] A. Liese, K. Seelbach, A. Buchholz, J. Haberland, in: Industrial Biotransformations, ed. A. Liese, K. Seelbach, C. Wandrey, Wiley-VCH, Weinheim, 2006, 2nd ed, pp 465 and pp 488.
[8] M. Wubbolts in: Enzyme Catalysis in Organic Synthesis, ed. K Drauz, H. Waldmann, Wiley-VCH, Weinheim, 2002, 2nd ed, pp 686-697.
[9] G. Agnihotri, H.-w. Liu, Bioorg. Med Chem. 2003, 11, 9-20.
[10] B. J. Bahnson, V. E. Anderson, G. A. Petsko, Biochem. 2002, 41, 2621-2629.
[11] A. F. Bell, Y. Feng, H. A. Hofstein, S. Parikh, J. Wu, M. J. Rudolph, C. Kisker, A. Whitty, P. J. Tonge, Chem. Biol. 2002, 9, 1247-1255.
[12] P. M Leonard, A. M Brzozowski, A. Lebedev, C. M. Marshall, D. J. Smith, C. S. Verma, N. J. Walton, G. Grogan, Acta Cryst. D 2006, 62, 1494-1501.
[13] P. M Leonard, C. M. Marshall, E. J. Dodson, N. J. Walton, G. Grogan, Acta Cryst. D 2004, 60, 2343-2345.
[14] A. Mitra, Y. Kitamura, M. J. Gasson, A. Narbad, A. J. Parr, J. Payne, M. J. C. Rhodes, C. Sewter, N. J. Walton, Arch. Biochem. Biophys. 1999, 365, 10-16.
[15] J. Jin, P. C. Oskam, S. K. Karmee, A. J. J. Straathof, U. Hanefeld, Chem. Commun. 2010, 46, 8588-8590.
[16] J. Jin, A. J. J. Straathof, M. W. H. Pinske, U. Hanefeld, Appl. Microbiol. Biotechnol. 2011, 89, 1831-1840.
[17] F. tenBrink, B. Schink, P. M. H. Kroneck, J. Bacteriol. 2011, 193, 1229-1236.
[18] R.-Z. Liao, F. Himo, ACS Catal. 2011, 1, 937-944.
[19] R.-Z. Liao, J.-G. Yu, F. Himo, Proc. Natl. Acad. Sci. USA 2010, 107, 22523-22527.
[20] G. B. Seiffert, G. M. Ullmann, A. Messerschmidt, B. Schink, P. M. H. Kroneck, O. Einsle, Proc. Natl. Acad. Sci. USA 2007, 104, 3073-3077.
[21] L. E. Bevers, M. W. H. Pinkse, P. D. E. M. Verhaert, W. R. J. Hagen, J. Bacteriol. 2009, 191, 5010-5012.
[22] A. Volkov, A. Liavonchanka, O. Kamneva, T. Fiedler, C. Goebel, B. Kreikemeyer, I. Feussner, J. Biol. Chem. 2010, 285, 10353-10361.
[23] C. S. Turbek, D. A. Simth, C. L. Schardl, FEMS Microbiol. Lett. 1992, 94, 187-190.
[24] J. A. Maresca, J. E. Graham, A. D. Pryant, Photosynth. Res. 2008, 97, 121-140.
[25] D. Brodkorb, M. Gottschall, R. Marmulla, F. Lüddeke, J. Harder, J. Biol. Chem. 2010, 285, 30436-30442.
[26] D. M. Smith, B. T. Golding, L. Radom, J. Am. Chem. Soc. 1999, 121, 5700-5704.
[27] T. Kamachi, T. Toraya, K. Yoshizawa, J. Am Chem. Soc. 2004, 126, 16207-16216.
[28] C. Wuensch, S. M. Glueck, J. Gross, D. Koszelewski, M. Schober, K. Faber, Org. Lett. 2012, 14, 1974-1977.
[29] H. Rodriguez, I. Angulo, B. de las Rivas, N. Campillo, J. A. Paez, R. Munoz, J. M. Mancheno, Proteins 2010, 78, 1662-1676.

## Claims

1. A process for the stereoselective hydration of a substrate compound using a decarboxylase.

2. The process according to claim 1, wherein the decarboxylase is selected from the group consisting of phenolic acid decarboxylase (PAD), ferulic acid decarboxylase (FDC), and β-coumaric acid decarboxylase (PDC).

3. The process according to claim 1 or 2, wherein the substrate is a compound of formula 1, 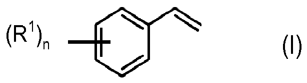 wherein
each R¹ is independently from one another selected from the group consisting of hydroxy, halogen, C₁₋₃alkoxy, C₁₋₃alkyl, C₁₋₃alkenyl, -NH₂, and
n is 0, 1, 2, 3 or 4.

4. The process according to claim 1 or 2, wherein the substrate is a compound of formula Ia, 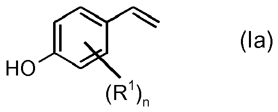 wherein
each R¹ is independently from one another selected from the group consisting of hydroxy, halogen, C₁₋₃alkoxy, C₁₋₃alkyl, C₁₋₃alkenyl, -NH₂, and
n is 0, 1, 2, 3 or 4.

5. A process for making a compound of formula II, comprising the step of 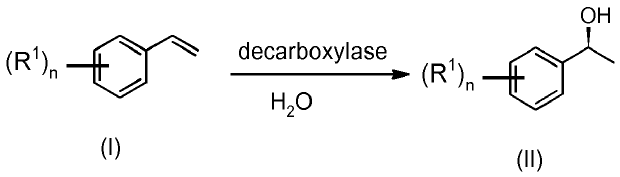 wherein
each R¹ is independently from one another selected from the group consisting of hydroxy, halogen, C₁₋₃alkoxy, C₁₋₃alkyl, C₁₋₃alkenyl, -NH₂, and
n is 0, 1, 2, 3 or 4.

6. A process for making a compound of formula IIa, comprising the step of 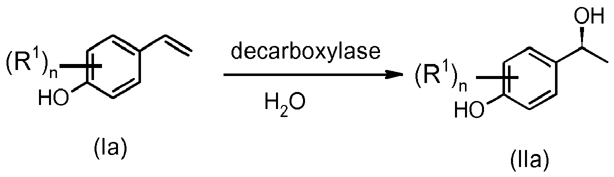 wherein
each R¹ is independently from one another selected from the group consisting of hydroxy, halogen, C₁₋₃alkoxy, C₁₋₃alkyl, C₁₋₃alkenyl, -NH₂, and
n is 0, 1, 2, 3 or 4.

7. The process according to claim 5 or 6, wherein a base is added.

8. The process according to claim 5 or 6, wherein the base is bicarbonate, or carbonate.

9. The process according to any one of claims 5 to 8, wherein compound II or compound Ila is obtained with at least 30%, preferably with at least 50%, more preferred with at least 70% conversion.

10. The process according to any one of claims 5 to 9, wherein compound II or compound Ila is obtained with at least 25%, preferably with at least 40%, more preferred with at least 55% enantiomeric excess (e.e.).

11. A process for manufacturing a hydrated compound, comprising the steps of:
i) suspending host cells which overexpress the decarboxylase in a buffer;
ii) adding the substrate compound;
iii) adding a base; and
iv) obtaining the hydrated compound.

12. A compound obtained in a process according to claim 11 for use in pharmaceutical, cosmetic and/or food industry.
